# EUROPEAN PATENT SPECIFICATION

(11) **EP 2 578 271 B1**
(45) Date of publication and mention of the grant of the patent: **23.08.2017**
(21) Application number: 12186968.9
(22) Date of filing: 02.10.2012
(51) Int. Cl.: A61N 5/10

(54) **Real-time tracking radiation therapy system**
System zur Verfolgung einer Strahlungstherapie in Echtzeit
Système de radiothérapie de suivi en temps réel

(30) Priority: 04.10.2011 JP 2011220122
(43) Date of publication of application: 10.04.2013
(73) Proprietor: Hitachi, Ltd., Tokyo 100-8280 (JP)
(72) Inventor: Umekawa, Toru, Tokyo, 100-8220 (JP); Fujii, Yusuke, Tokyo, 100-8220 (JP)
(74) Representative: MERH-IP Matias Erny Reichl Hoffmann Patentanwälte PartG mbB

(56) References cited:
- EP-A1- 1 430 835
- WO-A1-2005/009243
- WO-A1-2010/128431
- US-A1- 2006 193 435
- US-A1- 2010 189 218

## Description

### BACKGROUND OF THE INVENTION

### 1. Field of the Invention

The present invention relates to a radiotherapy system which irradiates a target lying in a subject with therapeutic radiation. The invention more particularly relates to a real-time tracking radiotherapy system which recognizes the position of a target in real time and irradiates the target with therapeutic radiation.

### 2. Description of the Related Art

A known moving object tracking radiotherapy system recognizes the position of a target in real time and applies the therapeutic radiation to the target so as to irradiate the target with therapeutic radiation in a concentrated manner and reduce the irradiation of each organ lying around the target therewith (refer to, for example, JP-2000-167072-A).

As a device for recognizing a target position, there is used a device for taking X-rays images from a plurality of directions and recognizing the target position from those X-ray images. Method for recognizing the position of a target include a method for directly recognizing the target and a method for indirectly recognizing the target from an surrogate marker located in the vicinity of the target. In the former method, a projection image of the target is prepared in advance as a template image and matched with each X-ray image, thereby computing a two-dimensional position of the target. Then, a three-dimensional position of the target is computed based on the two-dimensional position of the target, which has been obtained from the photographed image in each direction. In the latter method, a projection image of the surrogate marker is prepared in advance as a template image and matched with each photographed image, thereby computing a two-dimensional position of the surrogate marker. Then, a three-dimensional position of the surrogate marker is computed based on the two-dimensional position of the surrogate marker obtained from the photographed image in each direction. At this time, the relationship between the three-dimensional position of the surrogate marker and the three-dimensional position of the target has been captured in advance, and the target position is indirectly recognized.

According to the recognition of the above target position, such intercepted radiation as to perform radiation with the timing at which the position of the target and the applied position of radiation based on a treatment plan coincide with each other, or such pursuing radiation as to change the applied position of radiation in matching with the position of the target is carried out. Such moving object pursing enables accurate application of the radiation to an organ moved by respiration during therapy. Incidentally, in order to control the application of therapeutic radiation according to the motion of the target, X-ray taking is implemented with the frequency with which the motion of the target (or surrogate marker) can be captured.

Document US 2006/193435 A1 discloses a real-time tracking radiotherapy system in which the position of a target is detected based on taken X-ray images.

Document WO 2005/009243 A1 discloses to adjust a collimator such that a region of interest to be imaged by X-rays always includes an organ of a patient to be observed. The adjustment process is carried out automatically.

### SUMMARY OF THE INVENTION

The following problems however exist in the above related art. That is, in the above related art, an increase in the accuracy of application of the therapeutic radiation can be expected with the tracking of the moving object, while X-ray exposure occurs. Since lesser amount of X-ray exposure may be preferable, the imageing range is preferably reduced as long as the motion of the target or the surrogate marker can be captured. However, the range (in other words, passage or transit region) in which the target or the surrogate marker moves has heretofore roughly been estimated inclusive of errors due to day-to-day changes of state, and the photography range has been set larger than necessary.

The present invention has been made in view of the foregoing. An object of the present invention is to provide a moving object tracking radiotherapy system capable of appropriately setting a radiography range using a computed transit region of a target or an surrogate marker and achieving a reduction in the amount of X-ray exposure.
(1) In order to achieve the above object, the present invention provides a moving object tracking radiotherapy system including a therapeutic radiation irradiation device that applies therapeutic radiation to a target lying in a subject, at least two X-ray imaging devices each of which X-ray photographs the target or an surrogate marker adjacent to the target, a target position recognizing section that computes a two-dimensional position of the target or the surrogate marker as seen from one direction according to an image photographed by one of the X-ray imaging devices, computes a two-dimensional position of the target or the surrogate marker as seen from the other direction according to an image photographed by the other of the X-ray imaging devices, and computes a three-dimensional position of the target or the surrogate marker, based on these two-dimensional positions, a therapeutic radiation irradiation control section that controls the therapeutic radiation irradiation device, based on the three-dimensional position of the target or the surrogate marker, which has been computed by the target position recognizing section, thereby performing intercepted radiation or pursuing radiation to the target, each X-ray imaging device including an X-ray tube that irradiates the subject with X-rays, an X-ray detector that detects a two-dimensional dose distribution of the X-rays penetrated through the subject, and X-ray variable diaphragms each capable of restricting an irradiation range of the X-ray tube to thereby vary a radiography range, a transit region calculation section that computes a transit region of the target or the surrogate marker based on a history of the two-dimensional position of the target or the surrogate marker, which has been computed by the target position recognizing section, a radiography range setting section that displays a radiography range setting screen, the radiography range setting screen indicating the transit region of the target or the surrogate marker computed by the transit region calculation section or a minimum radiography range internally including and circumscribing the transit region of the target or the surrogate marker, and a maximum radiography range inherent in each of the X-ray imaging devices, indicating a radiography range to be set and input while being restricted between the minimum radiography range and the maximum radiography range, and setting a radiography range using the radiography range setting screen, and a diaphragm control section that controls the X-ray variable diaphragms of the X-ray imaging devices in accordance with the radiography range set by the radiography range setting section.
(2) In the above (1), preferably, the radiography range setting section adds a predetermined compensation region to an outer peripheral side of the transit region of the target or the surrogate marker and displays the compensation region added transit region on the photography range setting screen.
(3) In the above (2), preferably, the radiography range setting section restricts a radiography range to be set and input on the radiography range setting screen between the minimum radiography range which internally includes and circumscribes the transit region of the target or the surrogate marker and the predetermined compensation region, and the maximum radiography range.
(4) In order to achieve the above object, the present invention provides a moving object tracking radiotherapy system including a therapeutic radiation irradiation device that applies therapeutic radiation to a target lying in a subject, at least two X-ray imaging devices each of which X-ray photographs the target or an surrogate marker adjacent to the target, a target position recognizing section that computes a two-dimensional position of the target or the surrogate marker as seen from one direction according to an image taken by one of the X-ray imaging devices, computes a two-dimensional position of the target or the surrogate marker as seen from the other direction according to an image taken by the other of the X-ray imaging devices, and computes a three-dimensional position of the target or the surrogate marker, based on these two-dimensional positions, a therapeutic radiation irradiation control section that controls the therapeutic radiation irradiation device, based on the three-dimensional position of the target or the surrogate marker, which has been computed by the target position recognizing section, thereby performing intercepted radiation or pursuing radiation to the target, each X-ray imaging device including an X-ray tube that irradiates the subject with X-rays, an X-ray detector that detects a two-dimensional dose distribution of the X-rays penetrated through the subject, and X-ray variable diaphragms each capable of restricting an irradiation range of the X-ray tube to thereby vary a radiography range, a transit region calculation section that computes a transit region of the target or the surrogate marker, based on a history of the two-dimensional position of the target or the surrogate marker, which has been computed by the target position recognizing section, a radiography range setting section that adds a predetermined compensation region to an outer peripheral side of the transit region of the target or the surrogate marker, which has been computed by the transit region calculation section, and automatically sets a radiography range which internally includes and circumscribes the transit region of the target or the surrogate marker and the predetermined compensation region, and a diaphragm control section that controls the X-ray variable diaphragms of the X-ray imaging devices in accordance with the radiography range set by the radiography range setting section.
(5) In the above (1-4), preferably, the transit region calculation section computes a transit region of the target or the surrogate marker, based on a transit region at the time that a prestored projection image of the target or the surrogate marker is moved along the history of the two-dimensional position of the target or the surrogate marker.

### BRIEF DESCRIPTION OF THE DRAWINGS

These and other features, objects and advantages of the present invention will become more apparent from the following description when taken in conjunction with the accompanying drawings wherein:
FIG. 1 is a schematic diagram showing a configuration of a moving object tracking radiotherapy system according to a first embodiment of the present invention;
FIG. 2 is a block diagram illustrating functional configurations of a target position recognizing device and a radiography range setting device in the first embodiment of the present invention along with related devices;
FIG. 3 is a diagram showing a schematic structure of an X-ray variable diaphragm of an X-ray imaging device in the first embodiment of the present invention and showing a radiography range of the X-ray imaging device;
FIG. 4 is a flow chart showing the contents of control processing by the radiography range setting device in the first embodiment of the present invention;
FIG. 5 is a diagram showing a radiography range setting screen in the first embodiment of the present invention;
FIG. 6 is a diagram illustrating a radiography range setting screen in one modification of the present invention;
FIG. 7 is a block diagram illustrating functional configurations of a target position recognizing device and a radiography range setting device in a second embodiment of the present invention along with related devices; and
FIG. 8 is a flow chart showing the contents of control processing by the radiography range setting device in the second embodiment of the present invention.

### DESCRIPTION OF THE PREFERRED EMBODIMENTS

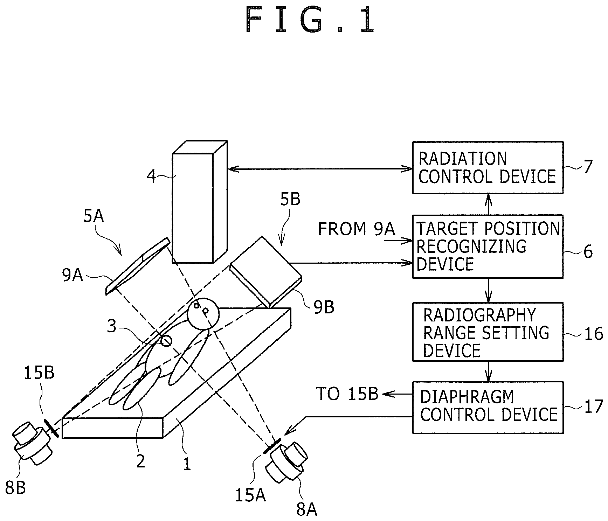

A first embodiment of the present invention will be explained with reference to FIGS. 1 through 5.

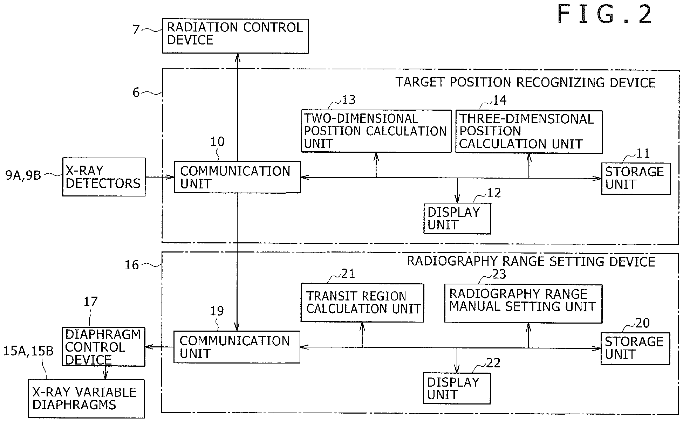

FIG. 1 is a schematic diagram showing a configuration of a moving object tracking radiotherapy system according to the present embodiment. FIG. 2 is a block diagram showing functional configurations of a target position recognizing device and a radiography range setting device in the present embodiment along with related devices.

The moving object tracking radiotherapy system includes a therapeutic radiation irradiation device 4, X-ray imaging devices 5A and 5B, a target position recognizing device 6, and a radiation control device 7. Specifically, the therapeutic radiation irradiation device 4 radiates therapeutic radiation (protons, for example) onto a target 3 lying in a subject 2 lying on a bed 1, the X-ray imaging devices 5A and 5B X-ray photograph the target 3 from a plurality of directions, the target position recognizing device 6 recognizes in real-time the position of the target 3 from each of images raken by the X-ray imaging devices 5A and 5B, and the radiation control device 7 which controls the therapeutic radiation irradiation device 4, based on the position of the target 3, which has been recognized by the target position recognizing device 6.

The X-ray imaging device 5A includes an X-ray tube 8A which irradiates the subject 2, an X-ray detector 9A, and an unillustrated signal processing circuit. Specifically, the X-ray tube 8A irradiates the subject 2 with X-rays from a first direction, the X-ray detector 9A detects a two-dimensional dose distribution of the X-rays radiated from the X-ray tube 8A and penetrated through the subject 2. The X-ray detector 9A has a plurality of detecting elements (specifically, e.g. semiconductor devices or the like which convert radiation into electrical charges) disposed on a two-dimensional basis and outputs analog signals detected therefrom. The signal processing circuit processes the analog signals output from the X-ray detector 9A to generate data of X-ray penetration images and transmits the same to the target position recognizing device 6. Incidentally, the radiography of the X-ray imaging device 5A is performed at a frequency (e.g., 30 kHz or so) enough to capture the motion of the target 3.

Likewise, the X-ray imaging device 5B includes an X-ray tube 8B, an X-ray detector 9B, and an unillustrated signal processing circuit. Specifically, the X-ray tube 8B irradiates the subject 2 with X-rays from a second direction (direction orthogonal to the first direction in the present embodiment), the X-ray detector 9B detects a two-dimensional dose distribution of the X-rays radiated from the X-ray tube 8B and transmitted through the subject 2. The X-ray detector 9B has a plurality of detecting elements disposed on a two-dimensional basis and outputs analog signals detected therefrom. The signal processing circuit processes the analog signals output from the X-ray detector 9B to generate data of X-ray penetration images and transmits the same to the target position recognizing device 6. Incidentally, the radiography of the X-ray imaging device 5B is performed in sync with that of the X-ray imaging device 5A.

The target position recognizing device 6 includes a communication unit 10, a storage unit (memory) 11, a display unit (monitor) 12, a two-dimensional position calculation unit 13, and a three-dimensional position calculation unit 14. Specifically, the communication unit 10 communicates between the X-ray imaging devices 5A and 5B, the radiation control device 7 and the like, the storage unit (memory) 11 stores therein photographed images received from the X-ray imaging devices 5A and 5B, and a result of calculation operation and the like to be described later, the display unit (monitor) 12 displays thereon the taken images of the X-ray imaging devices 5A and 5B and the result of calculation operation or the like to be described later, the two-dimensional position calculation unit 13 computes a two-dimensional position of the target 3 as viewed from the image taking direction (first direction) of the X-ray imaging device 5B according to the taken image of the X-ray imaging device 5A and computes a two-dimensional position of the target 3 as seen from the image taking direction (second direction) of the X-ray imaging device 5B according to the taken image of the X-ray imaging device 5B, and the three-dimensional position calculation unit 14 computes a three-dimensional position of the target 3, based on those two-dimensional positions of the target 3.

A projection image of the target 3 in the image takeing direction of the X-ray imaging device 5A has been prepared and stored in advance in the storage unit 11 of the target position recognizing device 6 as a first template image. A projection image of the target 3 in the image takeing direction of the X-ray imaging device 5B has been prepared and stored in advance therein as a second template image. The two-dimensional position calculation unit 13 matches the taken image of the X-ray imaging device 5 and the first template image with each other to thereby compute a two-dimensional position of the target 3 as seen from the image taking direction of the X-ray imaging device 5A. The two-dimensional position calculation unit 13 matches the photographed image of the X-ray imaging device 5B and the second template image with each other to thereby compute a two-dimensional position of the target 3 as seen from the image taking direction of the X-ray imaging device 5B. Specifically, similarity (e.g., normalization correlation coefficient) is computed by comparing the taken image and each template image while moving the two, and the position (matching position) where the similarity is the highest is assumed to be the two-dimensional position of the target 3. The so-computed two-dimensional position of target 3 is associated with its corresponding photographed image and stored in the storage unit 11.

The three-dimensional position calculation unit 14 of the target position recognizing device 6 backprojects the two-dimensional position (projection position) of the target 3 as seen from the image taking direction of each of the X-ray imaging devices 5A and 5B to thereby compute a three-dimensional position (projection position) of the target 3. The so-computed three-dimensional position of target 3 is to be displayed on the display unit 12 together with the corresponding photographed images of the X-ray imaging devices 5A and 5B. Thus, an operator is able to confirm the position of the target 3 in real time. Incidentally, the time taken for the matching processing can also be shortened by designating a target search region at each photographed image displayed on the display unit 12.

The computed three-dimensional position of target 3 is stored in the storage unit 11 and transmitted to the radiation control device 7. The radiation control device 7 controls the therapeutic radiation irradiation device 4 based on the three-dimensional position of the target 3 received from the target position recognizing device 6 to perform intercepted radiation (specifically, it means that the radiation is carried out with the timing at which the position of the target 3 and the applied position of radiation based on a therapy plan coincide with each other within a predetermined allowable range) or pursuing radiation (specifically, it means that the applied position of radiation is changed in matching with the position of the target 3).

Here, as prominent features of the present embodiment, the X-ray imaging device 5A has an X-ray variable diaphragm 15A capable of restricting a radiation range of the X-ray tube 8A and thereby varying a radiography range, whereas the X-ray imaging device 5B has an X-ray variable diaphragm 15B capable of restricting a radiation range of the X-ray tube 8B and thereby varying a radiography range. The moving object tracking radiotherapy system includes a radiography range setting device 16 which manually sets the radiography ranges of the X-ray imaging devices 5A and 5B, and a diaphragm control device 17 which drives and controls the X-ray variable diaphragms 15A and 15B according to the radiography ranges set by the radiography range setting device 16.

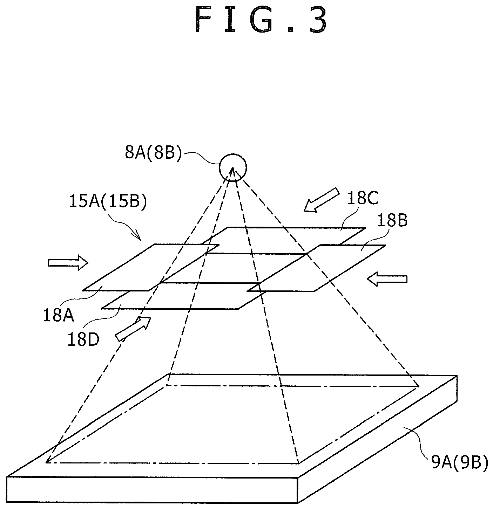

As shown in FIG. 3, for example, the X-ray variable diaphragm 15A has four diaphragm blades 18A through 18D which form a square slit (aperture) through which X-rays from the X-ray tube 8A pass, and a stepping motor (not shown) which moves the diaphragm blades 18A through 18D in directions (directions indicated by open arrows in FIG. 3) substantially orthogonal to the X-ray radiation direction. The radiation range (radiography range) becomes maximum in the arrangement of the diaphragm blades 18A through 18D shown in FIG. 3, for example, and the radiation range (radiography range) is reduced with the inward movement of the diaphragm blades 18A through 18D. Incidentally, since the structure of the X-ray variable diaphragm 15B is also similar to that of the X-ray variable diaphragm 15A, its description will be omitted.

The radiography range setting device 16 includes a communication unit 19, a storage unit 20 (memory), a target transit region calculation unit 21, and a radiography range manual setting unit 23. Specifically, the communication unit 19 communicates between the target position recognizing device 6 and the diaphragm control device 17, the storage unit 20 (memory) stores therein the histories of the two-dimensional positions of the target 3 as seen from the image taking directions of the X-ray imaging devices 5A and 5B, have been received from the target position recognizing device 6, and a result of calculation and the like to be described later, the target transit region calculation unit 21 computes a transit region of the target 3 as seen from the image taking direction of the X-ray imaging device 5A based on the history of the two-dimensional position of the target 3 as seen from the image taking direction of the X-ray imaging device 5A and computes a transit region of the target 3 as seen from the image taking direction of the X-ray imaging device 5B, based on the history of the two-dimensional position of the target 3 as seen from the image taking direction of the X-ray imaging device 5B, and the radiography range manual setting unit 23 causes a display unit (monitor) 22 to display a radiography range setting screen (to be described in detail later) for the X-ray imaging device 5A or 5B, which is indicative of the transit region of the target 3 or the like and a radiography range to be set and input, and manually sets the radiography ranges of the X-ray imaging devices 5A and 5B using the radiography range setting screen.

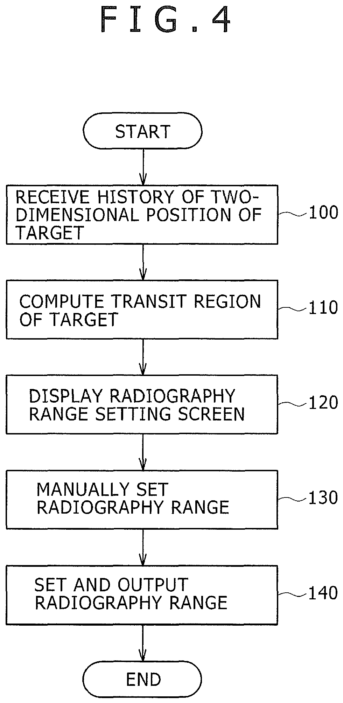

A procedure for control of the radiography range setting device 16 being an essential part of the present embodiment will next be described using FIG. 4. FIG. 4 is a flow chart showing the contents of control processing by the radiography range setting device 16 in the present embodiment.

First, at Step 100, the histories of the two-dimensional positions of the target 3 as seen from the image taking directions of the X-ray imaging devices 5A and 5B, which have been stored in the storage unit 11 of the target position recognizing device 6, are transmitted according to a command for the setting and change of the radiography range by an operation input of an operator, for example. The radiography range setting device 16 receives the histories therein and stores the same in the storage unit 20.

Thereafter, the radiography range setting device 16 proceeds to Step 110, where the transit region calculation unit 21 of the radiography range setting device 16 computes a transit region of the target 3 as viewed from the image taking direction of the X-ray imaging device 5A based on the history of the two-dimensional position of the target 3 as seen from the image taking direction of the X-ray imaging device 5A, and computes a transit region of the target 3 as seen from the image taking direction of the X-ray imaging device 5B based on the history of the two-dimensional position of the target 3 as seen from the image taking direction of the X-ray imaging device 5B. Described in detail, the above-described first and second template images have been stored in the storage unit 20 of the radiography range setting device 16. The transit region calculation unit 21 computes a transit region taken when the first template image (or the second template image) is moved along the history of the two-dimensional position of the target 3 as seen from the image taking direction of the X-ray imaging device 5A (or 5B and subsequent adaptation is the same as in parentheses), as a transit region of the target 3 as seen form the image taking direction of the X-ray imaging device 5A (or 5B). The computed transit region of target 3 is stored in the storage unit 20.

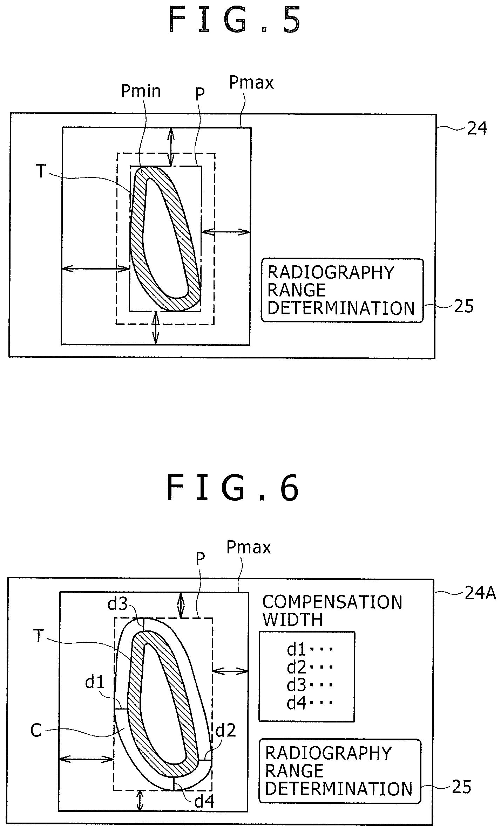

Thereafter, the radiography range setting device 16 proceeds to Step S120, where the radiography range manual setting unit 23 of the radiography range setting device 16 creates such a radiography range setting screen 24 for X-ray imaging device 5A (5B) as shown in FIG. 5, for example and displays it on the display unit 22.

The radiography range setting screen 24 indicates its corresponding transit region T of target 3 and indicates the minimum radiography range Pmin which internally includes the transit region T of the target 3 and circumscribes the transit region T thereof. The radiography range manual setting unit 23 computes the minimum radiography range Pmin as a position where the blades of the X-ray variable diaphragm are moved to narrow an aperture from the maximum aperture position and thereby the sides of the radiography region contact the transit region T. The minimum radiography range Pmin indicates the maximum radiography range Pmax inherent in the X-ray imaging device 5A (or 5B), i.e., a radiography range in which the aperture of the X-ray variable diaphragm 15A (or 15B) is maximum. The minimum radiography range Pmin indicates a radiography range P to be set and input while being restricted between the minimum radiography range Pmin and the maximum radiography range Pmax. Incidentally, each of the minimum radiography range Pmin, the maximum radiography range Pmax and the radiography range P indicates a shape (square shape in the present embodiment) corresponding to the X-ray variable diaphragm. Since the transit region T of the target 3 and the minimum radiography range Pmin indicate the restriction of the radiography range P, it is not necessary to show either one of the transit region T of the target 3 and the minimum radiography range Pmin.

The radiography range setting device 16 proceeds to Step 130, where the radiography range P on the radiography range setting screen 24 is changed according to the operation input of the operator. Described in detail, for example, when the state of an initial display of the radiography range P is of the minimum radiography range Pmin and any one of the four sides that configure the radiography range P is selected, the selected one side can be moved between the minimum radiography range Pmin and the maximum radiography range Pmax. Incidentally, its moving pitch has been set in advance in a correlation with the moving pitch of each of the diaphragm blades of the variable diaphragm 15A (or 15B). When the plural blades of the variable diaphragm are moved in cooperation with one another, each cooperating side is moved according to the operation of the selected one side of the radiography range. When each cooperating side has reached Pmin or Pmax, the movement of each side is stopped. When a radiography range determination button 25 on the radiography range setting screen 24 is operated, the radiography range manual setting unit 23 sets and stores the radiography range P being in display in the storage unit 20.

Thereafter, the radiography range setting device 16 proceeds to Step 140, where it transmits the radiography range P (or parameters corresponding thereto) set and stored in the storage unit 20 to the diaphragm control device 17. Correspondingly, the diaphragm control device 17 drives and controls the X-ray variable diaphragm 15A (or 15B) .

Operations and operative effects of the present embodiment will next be described.

When the operator inputs a command for X-ray taking through operation with a view toward performing X-ray taking for a predetermined time (time greater than or equal to one cycle when the target 3 moves periodically, for example) before the start of treatment (i.e., before therapeutic radiation is applied), for example, the X-ray imaging devices 5A and 5B perform taking X-rays of the target 3. At this time, each of the radiography ranges of the X-ray imaging devices 5A and 5B is controlled to the initial value (specifically, a radiography range set greater than necessary to assuredly capture the motion of the target 3) set and stored in the storage unit 20 of the radiography range setting device 16 in advance.

When the operator inputs a command for the setting and change of a radiography range through operation after the completion of taking X-rays , for example, the radiography range setting device 16 receives and stores the histories of two-dimensional positions of the target 3 as seen from the image taking directions of the X-ray imaging devices 5A and 5B and computes transit regions of the target 3 as seen from the image taking directions of the X-ray imaging devices 5A and 5B based on the histories. A radiography range setting screen 24 for the X-ray imaging device 5A or 5B is displayed and the radiography range of the X-ray imaging device 5A or 5B is manually set using the radiography range setting screen 24. The radiography range setting screen 24 indicates the transit region T (and minimum radiography range Pmin) of the target 3 and the maximum radiography range Pmax along with the set and input radiography range P. This enables the operator to easily and accurately set the radiography range of the X-ray imaging device 5A or 5B. It is thus possible to reduce the amount of X-ray exposure at subsequent therapy.

Incidentally, although the first embodiment has explained as an example, where the history of each two-dimensional position of the target 3, which has been stored in the storage unit 11 of the target position recognizing device 6, is transmitted according to the command for the setting and change of the radiography range, and the radiography range setting device 16 receives the history and stores the same in the storage unit 20, the present invention is not limited to this. That is, during the taking X-rays, the two-dimensional position of the target 3, which has been computed by the two-dimensional position calculation unit 13 of the target position recognizing device 6 is transmitted in real time. The radiography range setting device 16 may receive the two-dimensional position of the target 3 and store the same in the storage unit 20.

Although the first embodiment has explained as an example, where taking of the X-ray is performed before the start of therapy (i.e., before the start of application of the therapeutic radiation), the transit region of the target 3 is computed based on the two-dimensional position of the target 3 obtained upon the X-ray photographing, the radiography range setting screen 24 indicating the transit region of the target 3 and the like is displayed, and the radiography range is manually set using the radiography range setting screen 24, the present invention is not limited to this. That is, for example, the transit region of the target 3 is computed based on the two-dimensional position of the target 3 obtained, for example, during therapy or by temporarily interrupting therapy and taking X-rays during therapy (i.e., during the irradiation of therapeutic radiation). A radiography range setting screen indicating the transit region of the target 3 and the like may be displayed. At this time, the radiography range on the radiography range setting screen 24 is caused to display the previous setting state, thereby making it possible to confirm the relationship between the transit region of the target 3 and the radiography range during therapy. The radiography range may be set and changed as the case may be.

Although not described in particular in the first embodiment, a radiography range setting screen 24A may be displayed with the addition of a compensation region C to the outer peripheral side of a target transit region 25 as in a modification shown in FIG. 6, for example. The compensation region C is equivalent to one obtained by estimating a change in the transit region T of the target 3 with a change in the state of the subject 2 or the like. This is created based on compensation widths d1 through d4 set in advance in association with variable directions (left, right, upper and lower directions in the drawing) of the radiography range P, for example. In such a modification, the state of the initial display of the radiography range P may be defined as a radiography range which internally includes and circumscribes the transit region T of the target 3 as with the first embodiment, but instead, the state of the initial display of the radiography range P may be defined as a radiography range which internally includes and circumscribes the transit region T of the target 3 and the compensation region C (refer to FIG. 6). The minimum radiography range Pmin may be set as a radiography range which internally includes and circumscribes the transit region T of the target 3 as with the first embodiment, but instead, the radiography range P to be set and input may be limited to the minimum radiography range Pmin as a radiography range which internally includes and circumscribes the transit region T of the target 3, and the compensation region C. In such a modification, the radiography range can appropriately be set without depending on the ability and experiences of the operator as compared with the first embodiment (i.e., the case where it does not indicate the compensation region C). Particularly when the minimum radiography range Pmin is set as the radiography range which internally includes and circumscribes the transit region T of the target 3 and the compensation region C, the probability that the target 3 will depart from the radiography range is lowered, by extension, the probability that the irradiation of therapeutic radiation will be interrupted is lowered.

Although the first embodiment has explained as an example where the radiography ranges of the X-ray imaging devices 5A and 5B are individually set, the present invention is not limited to this. The radiography ranges of the X-ray imaging devices 5A and 5B may simultaneously be set in liaison with each other. Specifically, the direction orthogonal to the image taking direction (first direction) of the X-ray imaging device 5A and the image taking direction (second direction) of the X-ray imaging device 5B becomes a common direction in the radiography range of the X-ray imaging device 5A and the radiography range of the X-ray imaging device 5B. Therefore, the radiography range setting screen may be displayed in such a manner that the length of the radiography range of the X-ray imaging device 5A and the length of the radiography range of the X-ray imaging device 5B in the common direction are in line with each other. Thus, the radiography range can be set easier and more appropriately.

Although the first embodiment has explained as an example, the configuration in which each of the X-ray variable diaphragms 15A and 15B has the four diaphragm blades 18A through 18D that form the square-shaped slit and the stepping motor which moves the diaphragm blades 18A through 18D in the directions substantially orthogonal to the X-ray radiation direction, the present invention is not limited to this. That is, the number of the diaphragm blades is not limited to four, but may be less than or equal to three or greater than or equal to five. The slit formed by the diaphragm blades is not limited to the square shape, but may be another polygonal or circular shape. For example, the diaphragm blades may be configured to move in the X-ray radiation direction. The radiography range setting device 16 may display the shape of the radiography range corresponding to the X-ray variable diaphragm or such a radiography range setting screen as to set the radiography range by a variable method. Even in such a case, advantageous effects similar to the above can be obtained.

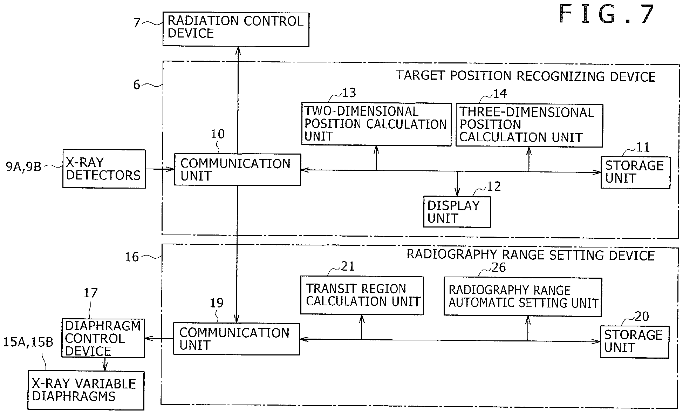

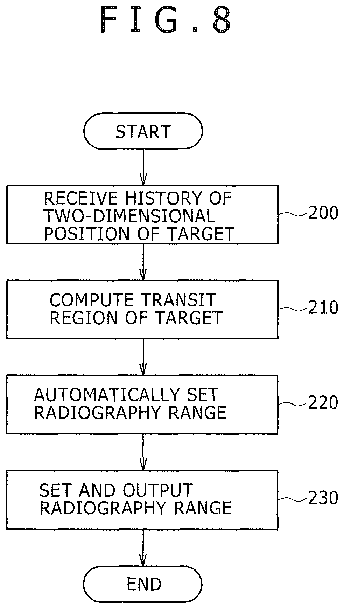

A second exemplary embodiment will be described with reference to FIGS. 7 and 8. The present embodiment is an embodiment in which the radiography ranges of X-ray imaging devices 5A and 5B are automatically set. Incidentally, in the present embodiment, the same reference numerals are respectively attached to components equivalent to those in the first embodiment, and their description will be omitted as appropriate.

FIG. 7 is a block diagram illustrating functional configurations of a target position recognizing device and a radiography range setting device in the present embodiment along with related devices.

In the present embodiment, the radiography range setting device 16A includes a communication unit 19, a storage unit 20 (memory), a target transit region calculation unit 21, and a radiography range automatic setting unit 26. Specifically, the communication unit 19 communicates between a target position recognizing device 6 and a diaphragm control device 7 or the like, the storage unit 20 (memory) stores therein a history of a two-dimensional position of a target 3 as seen from the direction of radiography of each of X-ray imaging devices 5A and 5B, and a result of calculation operation and the like to be described later, all of which have been received from the target position recognizing device 6, the target transit region calculation unit 21 computes a transit region of the target 3 as seen from the image taking direction of the X-ray imaging device 5A, based on the history of the two-dimensional position of the target 3 as seen from the image taking direction of the X-ray imaging device 5A and computes a transit region of the target 3 as seen from the image taking direction of the X-ray imaging device 5B, based on the history of the two-dimensional position of the target 3 as seen from the image taking direction of the X-ray imaging device 5B, and the radiography range automatic setting unit 26 automatically sets a radiography range, based on the transit region of the target 3.

A procedure for control of the radiography range setting device 16A being an essential part of the present embodiment will next be described using FIG. 8. FIG. 8 is a flow chart showing the contents of control processing by the radiography range setting device 16A in the present embodiment.

First, at Step 200, the histories of the two-dimensional positions of the target 3 as seen from the image taking directions of the X-ray imaging devices 5A and 5B, which have been stored in the storage unit 11 of the target position recognizing device 6, are transmitted according to a command for the setting and change of a radiography range by an operation input of an operator, for example. The radiography range setting device 16A receives the histories therein and stores the same in the storage unit 20.

Thereafter, the radiography range setting device 16A proceeds to Step 210, where the transit region calculation unit 21 of the radiography range setting device 16A computes a transit region of the target 3 as viewed from the image taking direction of the X-ray imaging device 5A, based on the history of the two-dimensional position of the target 3 as seen from the image taking direction of the X-ray imaging device 5A, and computes a transit region of the target 3 as seen from the image taking direction of the X-ray imaging device 5B, based on the history of the two-dimensional position of the target 3 as seen from the image taking direction of the X-ray imaging device 5B. The transit region calculation unit 21 stores those regions in the storage unit 20.

Afterwards, the radiography range setting device 16A proceeds to Step 220, where the radiography range automatic setting unit 26 of the radiography range setting device 16A adds a predetermined compensation region to the outer peripheral side of the transit region of the target 3 as seen from the image taking direction of the X-ray imaging device 5A, automatically sets a radiography range (refer to the above radiography range P in FIG. 6) of the X-ray imaging device 5A, which internally includes these transit and compensation regions of the target 3 and circumscribes them, and stores the same in the storage unit 20. The radiography range automatic setting unit 26 automatically sets a radiography range (refer to the above radiography range P in FIG. 6) of the X-ray imaging device 5B which internally includes these transit and compensation regions of the target 3 and circumscribes them, and stores them in the storage unit 20.

Thereafter, the radiography range setting device 16A proceeds to Step 230, where the radiography range P (or parameters corresponding thereto) set and stored in the storage unit 20 is transmitted to the diaphragm control device 17. Correspondingly, the diaphragm control device 17 drives and controls an X-ray variable diaphragm 15A (or 15B) .

Operations and operative effects of the present embodiment will next be described.

When the operator inputs a command for taking X-rays through operation with a view toward taking X-rays for a predetermined time (time greater than or equal to one cycle when the target 3 moves periodically, for example) before the start of therapy (i.e., before therapeutic radiation is applied), for example, the X-ray imaging devices 5A and 5B take X-ray of the target 3. At this time, each of the radiography ranges of the X-ray imaging devices 5A and 5B is controlled to the initial value (specifically, a radiography range set greater than necessary to assuredly capture the motion of the target 3) set and stored in the storage unit 20 of the radiography range setting device 16A in advance.

When the operator inputs a command for the setting and change of a radiography range through operation after the completion of taking X-rays, for example, the radiography range setting device 16A receives and stores the history of a two-dimensional position of the target 3 as seen from the image taking direction of each of the X-ray imaging devices 5A and 5B and computes a transit region of the target 3 as seen from the image taking direction of each of the X-ray imaging devices 5A and 5B, based on the history. The radiography range setting device 16A adds a predetermined compensation region to the outer peripheral side of the transit region of the target 3 as seen from the image taking direction of each of the X-ray imaging devices 5A and 5B and automatically sets the radiography range of each of the X-ray imaging devices 5A and 5B, which internally includes and circumscribes these transit and compensation regions of the target 3. It is therefore possible to easily and accurately set the radiography ranges of the X-ray imaging devices 5A and 5B. It is thus possible to reduce the amount of X-ray exposure at subsequent therapy.

Incidentally, although the second embodiment has explained as an example, where the history of each two-dimensional position of the target 3, which has been stored in the storage unit 11 of the target position recognizing device 6 is transmitted according to the command for the setting and change of the radiography range, and the radiography range setting device 16A receives the history and stores the same in the storage unit 20, the present disclosure is not limited to this. That is, during the taking X-rays, the two-dimensional position of the target 3, which has been computed by a two-dimensional position calculation unit 13 of the target position recognizing device 6, is transmitted in real time. The radiography range setting device 16A may receive the two-dimensional position of the target 3 and store the same in the storage unit 20.

Although the second embodiment has described as an example, where the taking X-rays is performed before the start of therapy (i.e., before the start of application of the therapeutic radiation), the transit region of the target 3 is computed based on the two-dimensional position of the target 3 obtained upon the taking X-rays, and the radiography range is automatically set using the transit region of the target 3, the present disclosure is not limited to this. That is, for example, the transit region of the target 3 is computed based on the two-dimensional position of the target 3 obtained during treatment or by temporarily interrupting treatment and performing taking X-rays during therapy (i.e., during the application of therapeutic radiation). The radiography range may automatically be set based on the transit region of the target 3.

Although the second embodiment has described as an example, where the radiography range setting device 16A does not have the display unit (monitor), the present disclosure is not limited to this. The radiography range setting device 16A may include the display unit. And the radiography range setting device 16A may indicate, for example, the transit region (and compensation region) of the target 3 and display a radiography range confirmation screen indicative of an automatically set radiography range.

Although each of the first and second embodiments has explained as an example, where the target position recognizing device 6, the radiation control device 7, the radiography range setting device 16 or 16A and the diaphragm control device 17 are configured as separate parts, the present invention is not limited to this, but it is needless to say that any of those may be taken as an integral configuration. Although the first and second embodiments have described as an example, where the two X-ray imaging devices 5A and 5B are provided, the present invention is not limited to this, but it is needless to say that three or more X-ray imaging devices are provided.

Incidentally, although the moving object tracking radiotherapy system in which the X-ray imaging devices 5A and 5B take X-ray images of the target 3 in the subject 2 and the target position recognizing device 6 directly recognizes the target 3 has been described above by way of example as a target for application of the present invention, the present invention is not limited to this. That is, there may be adopted a moving object tracking radiotherapy system in which the X-ray imaging devices 5A and 5B image an surrogate marker near the target 3 and the target position recognizing device 6 indirectly recognizes the target 3 from the surrogate marker. This will be described below in detail.

In this moving object tracking radiotherapy system, a projection image of the surrogate marker in the image taking direction of the X-ray imaging device 5A has been prepared and stored in advance in the storage unit 11 of the target position recognizing device 6 as a first template image. A projection image of the alternative marker in the image taking direction of the X-ray imaging device 5B has been prepared and stored in advance as a second template image. The two-dimensional position calculation unit 13 matches a taken image of the X-ray imaging device 5A and the first template image with each other to thereby compute a two-dimensional position of the surrogate marker as seen from the image taking direction of the X-ray imaging device 5A. The two-dimensional position calculation unit 13 matches a image of the X-ray imaging device 5B and the second template image with each other to thereby compute a two-dimensional position of the surrogate marker as seen from the image taking direction of the X-ray imaging device 5B. The computed two-dimensional positions of surrogate marker are stored in the storage unit 11. A three-dimensional position calculation unit 14 of the target position recognizing device 6 backprojects the two-dimensional position (projection position) of the surrogate marker as seen from the image taking direction of each of the X-ray imaging devices 5A and 5B to compute a three-dimensional position (projection position) of the surrogate marker. The computed three-dimensional position of surrogate marker is displayed on the display unit 12 together with the images of the X-ray imaging devices 5A and 5B. The computed three-dimensional position of surrogate marker is stored in the storage unit 11 and transmitted to the radiation control device 7. At this time, the relationship between the three-dimensional position of the surrogate marker and its corresponding three-dimensional position of the target 3 has been captured in advance. The radiation control device 7 controls the therapeutic radiation irradiation device 4 based on the three-dimensional position of the surrogate marker received from the target position recognizing device 6 to thereby perform intercepted radiation or pursuing radiation to the target.

When the present invention is applied to such a moving object tracking radiotherapy system, a radiography range setting device computes a transit region of an surrogate marker based on the history of a two-dimensional position of the surrogate marker. As with the first embodiment (and modification), for example, a radiography range setting screen indicative of the transit region of the surrogate marker, the minimum radiography range and the maximum radiography range or the like, and a radiography range to be set and input is displayed, A radiography range is manually set using the radiography range setting screen. Alternatively, for example, the radiography range is automatically set based on the transit region of the surrogate marker as with the second embodiment. Even in such a case, advantageous effects similar to the above can be obtained.

While we have shown and described several embodiments in accordance with our invention, it should be understood that disclosed embodiments are susceptible of changes and modifications without departing from the scope of the invention. Therefore, we do not intend to be bound by the details shown and described herein but intend to cover all such changes and modifications within the ambit of the appended claims.

## Claims

1. A real-time tracking radiotherapy system comprising:
a therapeutic radiation irradiation device (4) adapted to apply therapeutic radiation to a target (3) lying in a subject (2);
at least two X-ray imaging devices (5A; 5B) each of which being adapted to take an X-ray image of the target (3) or an surrogate marker adjacent to the target (3);
target position recognizing means (6) adapted to compute a two-dimensional position of the target (3) or the surrogate marker as seen from one direction according to an image taken by one of the X-ray imaging devices (5A; 5B), compute a two-dimensional position of the target (3) or the surrogate marker as seen from the other direction according to an image taken by the other of the X-ray imaging devices (5A; 5B), and compute a three-dimensional position of the target (3) or the surrogate marker, based on these two-dimensional positions;
therapeutic radiation irradiation control means (7) adapted to control the therapeutic radiation irradiation device (4), based on the three-dimensional position of the target (3) or the surrogate marker, which has been computed by the target position recognizing means (6), to thereby perform intercepted radiation or pursuing radiation to the target (3);
each X-ray imaging device (5A; 5B) including an X-ray tube adapted to irradiate the subject (2) with X-rays, an X-ray detector (9A; 9B) adapted to detect a two-dimensional dose distribution of the X-rays penetrated through the subject (2), and X-ray variable diaphragms (15A; 15B) each capable of restricting an irradiation range of the X-ray tube to thereby vary a radiography range;
the system being **characterised by** transit region calculate means (21) adapted to compute a transit region of the target or the surrogate marker based on a history of the two-dimensional position of the target or the surrogate marker, which has been computed by the target position recognizing means (6); the transit region being the range in which the target or the surrogate marker moves; radiography range setting means (16) adapted to display a radiography range setting screen (24), the radiography range setting screen (24) indicating the transit region of the target (3) or the surrogate marker computed by the transit region calculate means (21) or a minimum radiography range internally including and circumscribing the transit region of the target (3) or the surrogate marker, and a maximum radiography range inherent in each of the X-ray imaging devices (5A; 5B), the radiography range setting screen (24) further indicating a radiography range to be set and input while being restricted between the minimum radiography range and the maximum radiography range, the radiography range setting means further adapted to accept an operation input of an operator using the radiography range setting screen to set the radiography range and diaphragm control means (17) adapted to control the X-ray variable diaphragms (15A; 15B) of the X-ray imaging devices (5A; 5B) in accordance with the radiography range set by the radiography range setting means (16).

2. The real-time tracking radiotherapy system according to claim 1,
wherein the radiography range setting means (16) is adapted to add a predetermined compensation region to an outer peripheral side of the transit region of the target (3) or the surrogate marker and display the compensation region added transit region on the radiography range setting screen (24).

3. The real-time tracking radiotherapy system according to claim 2,
wherein the radiography range setting means (16) is adapted to restrict a radiography range to be set and input on the radiography range setting screen (24) between the minimum radiography range which internally includes and circumscribes the transit region of the target (3) or the surrogate marker and the predetermined compensation region, and the maximum radiography range.

4. The real-time tracking radiotherapy system according to any one of claims 1 to 3, wherein the transit region calculate means (21) is adapted to compute a transit region of the target (3) or the surrogate marker, based on a transit region at the time that a prestored projection image of the target (3) or the surrogate marker is moved along the history of the two-dimensional position of the target (3) or the surrogate marker.

## Patentansprüche

1. Echtzeitnachverfolgungs-Strahlentherapiesystem, das Folgendes umfasst:
eine Vorrichtung (4) zur therapeutischen Bestrahlung, die ausgelegt ist, auf ein Ziel (3), das in einer Testperson (2) liegt, eine therapeutische Strahlung anzuwenden;
mindestens zwei Röntgenbildgebungsvorrichtungen (5A; 5B), wovon jede ausgelegt ist, ein Röntgenbild des Ziels (3) oder einer an das Ziel (3) angrenzenden Ersatzmarkierung aufzunehmen;
Zielpositionserkennungsmittel (6), die ausgelegt sind, bei Betrachtung aus einer Richtung entsprechend einem Bild, das durch eine der Röntgenbildgebungsvorrichtungen (5A; 5B) aufgenommen wird, eine zweidimensionale Position des Ziels (3) oder der Ersatzmarkierung zu berechnen, bei Betrachtung aus der anderen Richtung entsprechend einem Bild, das durch die andere der Röntgenbildgebungsvorrichtungen (5A; 5B) aufgenommen wird, eine zweidimensionale Position des Ziels (3) oder der Ersatzmarkierung zu berechnen, und auf der Basis dieser zweidimensionalen Positionen eine dreidimensionale Position des Ziels (3) oder der Ersatzmarkierung zu berechnen;
Mittel (7) zum Steuern der therapeutischen Bestrahlung, die ausgelegt sind, die Vorrichtung (4) zur therapeutischen Bestrahlung auf der Basis der dreidimensionalen Position des Ziels (3) oder der Ersatzmarkierung, die durch die Zielpositionserkennungsmittel (6) berechnet wurden, zu steuern, um dadurch eine aufgefangene Strahlung oder eine nachverfolgende Strahlung auf das Ziel (3) zu erzeugen;
wobei jede Röntgenbildgebungsvorrichtung (5A; 5B) eine Röntgenröhre, die ausgelegt ist, die Testperson (2) mit Röntgenstrahlen zu bestrahlen, einen Röntgenstrahlendetektor (9A; 9B), der ausgelegt ist, eine zweidimensionale Dosierungsverteilung der Röntgenstrahlen, die die Testperson (2) durchdrungen haben, zu detektieren, und veränderliche Röntgenstrahlenmembranen (15A; 15B), die jeweils einen Bestrahlungsbereich der Röntgenröhre einschränken können, um dadurch einen Röntgendurchleuchtungsbereich zu verändern, enthält;
wobei das System **gekennzeichnet ist durch**
Mittel (21) zum Berechnen des Übergangsbereichs, die ausgelegt sind, anhand einer Historie der zweidimensionalen Position des Ziels oder der Ersatzmarkierung, die durch die Zielpositionserkennungsmittel (6) berechnet wurde, einen Übergangsbereich des Ziels oder der Ersatzmarkierung zu berechnen;
wobei der Übergangsbereich der Bereich ist, in dem sich das Ziel oder die Ersatzmarkierung bewegen;
Mittel (16) zum Einstellen des Röntgendurchleuchtungsbereichs, die ausgelegt sind, einen Bildschirm (24) zum Einstellen des Röntgendurchleuchtungsbereichs anzuzeigen, wobei der Bildschirm (24) zum Einstellen des Röntgendurchleuchtungsbereichs den Übergangsbereich des Ziels (3) oder der Ersatzmarkierung, der durch die Mittel (21) zum Berechnen des Übergangsbereichs berechnet wird, oder einen minimalen Röntgendurchleuchtungsbereich, der intern den Übergangsbereich des Ziels (3) oder der Ersatzmarkierung enthält und umschreibt, und einen maximalen Röntgendurchleuchtungsbereich, der jeder der Röntgenbildgebungsvorrichtungen (5A; 5B) inhärent ist, anzeigf, wobei der Bildschirm (24) zum Einstellen des Röntgendurchleuchtungsbereichs ferner einen Röntgendurchleuchtungsbereich anzeigt, der einzustellen und einzugeben ist, während er zwischen dem minimalen Röntgendurchleuchtungsbereich und dem maximalen Röntgendurchleuchtungsbereich eingeschränkt ist, wobei die Mittel zum Einstellen des Röntgendurchleuchtungsbereichs ferner ausgelegt sind, eine Bedieneingabe einer Bedienperson anzunehmen, die den Bildschirm zum Einstellen des Röntgendurchleuchtungsbereichs verwendet, um den Röntgendurchleuchtungsbereich einzustellen; und
Membransteuermittel (17), die ausgelegt sind, die veränderlichen Röntgenstrahlenmembranen (15A; 15B) der Röntgenbildgebungsvorrichtungen (5A; 5B) in Übereinstimmung mit dem Röntgendurchleuchtungsbereich, der durch die Mittel (16) zum Einstellen des Röntgendurchleuchtungsbereichs eingestellt wird, zu steuern.

2. Echtzeitnachverfolgungs-Strahlentherapiesystem nach Anspruch 1,
wobei das Mittel (16) zum Einstellen des Röntgendurchleuchtungsbereichs ausgelegt ist, einen vorgegebenen Ausgleichbereich zu einer Außenumfangsseite des Übergangsbereichs des Ziels (3) oder der Ersatzmarkierung hinzuzufügen und den um den Ausgleichsbereich ergänzten Übergangsbereich auf dem Bildschirm (24) zum Einstellen des Röntgendurchleuchtungsbereichs anzuzeigen.

3. Echtzeitnachverfolgungs-Strahlentherapiesystem nach Anspruch 2,
wobei das Mittel (16) zum Einstellen des Röntgendurchleuchtungsbereichs ausgelegt ist, einen Röntgendurchleuchtungsbereich, der auf dem Bildschirm (24) zum Einstellen des Röntgendurchleuchtungsbereichs einzustellen und einzugeben ist, zwischen dem minimalen Röntgendurchleuchtungsbereich, der intern den Übergangsbereich des Ziels (3) oder der Ersatzmarkierung und den vorgegebenen Ausgleichsbereich enthält und umschreibt, und dem maximalen Röntgendurchleuchtungsbereich einzuschränken.

4. Echtzeitnachverfolgungs-Strahlentherapiesystem nach einem der Ansprüche 1 bis 3,
wobei das Mittel (21) zum Berechnen des Übergangsbereichs ausgelegt ist, einen Übergangsbereich des Ziels (3) oder der Ersatzmarkierung auf der Basis eines Übergangsbereichs zu der Zeit, zu der ein vorab gespeichertes Projektionsbild des Ziels (3) oder der Ersatzmarkierung entlang der Historie der zweidimensionalen Position des Ziels (3) oder der Ersatzmarkierung bewegt wird, zu berechnen.

## Revendications

1. Système de radiothérapie avec suivi en temps réel, comprenant :
un dispositif d'irradiation à rayonnement thérapeutique (4) adapté à appliquer un rayonnement thérapeutique à une cible (3) qui se trouve dans un sujet (2) ;
au moins deux dispositifs d'imagerie à rayons X (5A ; 5B) dont chacun est adapté à prendre une image en rayons X de la cible (3) ou d'un marqueur de substitution adjacent à la cible (3) ;
un moyen de reconnaissance de position de cible (6) adapté à calculer une position bidimensionnelle de la cible (3) ou du marqueur de substitution, telle que vue depuis une direction en accord avec une image prise par l'un des dispositifs d'imagerie à rayons X (5A ; 5B), à calculer une position bidimensionnelle de la cible (3) ou du marqueur de substitution telle que vue depuis l'autre direction en accord avec une image prise par l'autre des dispositifs d'imagerie à rayons X (5A ; 5B), et à calculer une position tridimensionnelle de la cible (3) ou du marqueur de substitution, sur la base de ces deux positions bidimensionnelles ;
un moyen de commande d'irradiation du rayonnement thérapeutique (7), adapté à commander le dispositif d'irradiation de rayonnement thérapeutique (4) sur la base de la position tridimensionnelle de la cible (3) ou du marqueur de substitution, qui a été calculée par le moyen de reconnaissance de position de cible (6), pour effectuer ainsi une interception du rayonnement ou une poursuite du rayonnement vers la cible (3) ;
chaque dispositif d'imagerie à rayons X (5A ; 5B) inclut un tube à rayons X adapté à irradier le sujet (2) avec des rayons X, un détecteur de rayons X (9A ; 9B) adapté à détecter une distribution de dose bidimensionnelle des rayons X qui ont pénétré à travers le sujet (2), et des diaphragmes variables à rayons X (15A ; 15B) qui sont capables chacun de restreindre une plage d'irradiation du tube à rayons et pour faire varier ainsi une plage de radiographie ;
le système étant **caractérisé par**
un moyen de calcul de région de transit (21) adapté à calculer une région de transit de la cible ou du marqueur de substitution sur la base d'un historique de la position bidimensionnelle de la cible ou du marqueur de substitution, qui a été calculée par le moyen de reconnaissance de position de cible (6) ;
la région de transit étant la plage dans laquelle se déplace la cible ou le marqueur de substitution ;
un moyen de fixation de plage de radiographie (16) adapté à afficher un écran de fixation de plage de radiographie (24), l'écran de fixation de plage de radiographie (24) indiquant la région de transit de la cible (3) ou du marqueur de substitution calculée par le moyen de calcul de région de transit (21), ou une plage de radiographie minimum qui inclut à l'intérieur et qui circonscrit la région de transit de la cible (3) ou du marqueur de substitution, et une plage de radiographie maximum inhérente dans chacun des dispositifs d'imagerie à rayons X (5A ; 5B), l'écran de fixation de plage de radiographie (24) indiquant en outre une plage de radiographie à fixer et à saisir, tout en étant restreinte entre la plage de radiographie minimum et la plage de radiographie maximum, le moyen de fixation de plage de radiographie étant en outre adapté à accepter une opération d'entrée d'un opérateur utilisant l'écran de fixation de plage de radiographie pour fixer la plage de radiographie ; et
un moyen de commande de diaphragme (17) adapté à commander les diaphragmes variables à rayons X (15A ; 15B) des dispositifs d'imagerie à rayons X (5A ; 5B) en accord avec la plage de radiographie fixée par le moyen de fixation de plage de radiographie (16).

2. Système de radiothérapie avec suivi en temps réel selon la revendication 1,
dans lequel le moyen de fixation de plage de radiographie (16) est adapté à ajouter une région de compensation prédéterminée sur un côté périphérique extérieur de la région de transit de la cible (3) ou du marqueur de substitution et à afficher la région de compensation ajoutée à la région de transit sur l'écran de fixation de plage de radiographie (24).

3. Système de radiothérapie avec suivi en temps réel selon la revendication 2,
dans lequel le moyen de fixation de plage de radiographie (16) est adapté à restreindre une plage de radiographie à fixer et à saisir sur l'écran de fixation de plage de radiographie (24) entre la plage de radiographie minimum qui inclut à l'intérieur et qui circonscrit la région de transit de la cible (3) ou du marqueur de substitution et la région de compensation prédéterminée, et la plage de radiographie maximum.

4. Système de radiothérapie avec suivi en temps réel selon l'une quelconque des revendications 1 à 3,
dans lequel le moyen de calcul de région de transit (21) est adapté à calculer une région de transit de la cible (3) ou du marqueur de substitution, sur la base d'une région de transit au moment auquel une image en projection préalablement mémorisée de la cible (3) ou du marqueur de substitution est déplacée le long de l'historique de la position bidimensionnelle de la cible (3) ou du marqueur de substitution.
